(19) Europäisches Patentamt
European Patent Office
Office européen des brevets

(11) EP 3 656 755 A1

(12) **EUROPEAN PATENT APPLICATION**
published in accordance with Art. 153(4) EPC

(43) Date of publication:
**27.05.2020 Bulletin 2020/22**

(21) Application number: **18835965.7**

(22) Date of filing: **26.01.2018**

(51) Int Cl.:
***C07C 51/43** (2006.01)* ***C07C 51/48** (2006.01)*
***C07C 59/01** (2006.01)*

(86) International application number:
**PCT/JP2018/002463**

(87) International publication number:
**WO 2019/016983 (24.01.2019 Gazette 2019/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **19.07.2017 PCT/JP2017/026068**

(71) Applicant: **Kobayashi Perfumery Co., Ltd.**
**Tokyo 103-0023 (JP)**

(72) Inventors:
- **SUGITA Naoki**
  **Tokyo 103-0023 (JP)**
- **HOSHI Toshio**
  **Tokyo 103-0023 (JP)**

(74) Representative: **Schön, Christoph**
**Dr. Schön, Neymeyr & Partner mbB**
**Bavariaring 26**
**80336 München (DE)**

# (54) METHOD FOR PRODUCING 3-HYDROXY-3-METHYLBUTANOIC ACID OR SALT THEREOF

(57) The purpose of the present invention is to provide a method for producing a 3-hydroxy-3-methylbutyrate or salt thereof, the method being capable of efficiently producing a highly pure 3-hydroxy-3-methylbutyrate or salt thereof. The highly pure 3-hydroxy-3-methylbutyrate or salt thereof can be efficiently produced by performing at least one of the following operations (a) to (d) on a solution containing 3-hydroxy-3-methylbutyrate and impurities exposed to environments of pH 6.0 or less. (a) Hypochlorous acid or salt thereof is added to the solution. (b) The solution is maintained at 40 to 200 °C for 30 minutes or more, provided that the solution contains hypochlorous acid or salt thereof. (c) Calcium salt is added to the solution, and the precipitated calcium 2,3-dihydroxy-3-methylbutanoate is separated from the liquid phase in which 3-hydroxy-3-methylbutyrate and/or the salt is dissolved. (d) Abase is added to the solution to separate the liquid phase in which the salt of 2,3-dihydroxy-3-methylbutanoic acid formed is dissolved and the liquid phase in which 3-hydroxy-3-methylbutyrate and/or salt thereof is dissolved.

EP 3 656 755 A1

## Description

### TECHNICAL FIELD

The present invention relates to a method for producing 3-hydroxy-3-methylbutyrate or salt thereof.

### BACKGROUND ART

3-hydroxy-3-methylbutyrate (also called "β-hydroxy-β-methylbutyric acid", "3-hydroxyisovaleric acid", "HMB" and others) is a metabolic product of leucine, which is an essential amino acid, and is a useful compound having functions such as reducing muscle tissue damage during exercise, increasing muscle mass and muscle strength, recovery and improving muscle tissue damage, improving the balance between muscle synthesis and degradation, maintaining muscle mass and muscle strength, reducing body fat, tightening and improving basal metabolism.

As a typical method for producing 3-hydroxy-3-methylbutyrate, a method utilizing an oxidation reaction of diacetone alcohol (DAA) with hypochlorite is widely known, and in recent years, a method of performing this oxidation reaction in a continuous process has been proposed (refer to Patent Document 1). As a method for purifying 3-hydroxy-3-methylbutyrate, a method including base neutralization to form a salt, cooling and crystallization, dissolution and acidification, and extraction has been proposed (refer to Patent Document 2).

### CITATION LIST

### PATENT DOCUMENT

Patent Document 1: JP 2014-525410 A
Patent Document 2: JP 2016-514733 A

### SUMMARY OF THE INVENTION

### PROBLEMS TO BE SOLVED BY THE INVENTION

The purpose of the present invention is to provide a method for producing 3-hydroxy-3-methylbutyrate or salt thereof, which is capable of efficiently preparing a highly pure 3-hydroxy-3-methylbutyrate or salt thereof.

### MEANS FOR SOLVING PROBLEMS

As a result of intensive investigations to solve the above problems, the inventors have found that highly pure 3-hydroxy-3-methylbutyrate or salt thereof can be produced efficiently by performing a specific operation to a solution containing 3-hydroxy-3-methylbutyrate and impurities, thereby completing the present invention. That is, the present invention is as follows.

(1) A method for producing of 3-hydroxy-3-methylbutyrate or salt thereof comprising a preparation step for the preparation of a solution containing 3-hydroxy-3-methylbutyrate and impurities and a treatment step for reducing or eliminating the impurities in the solution, wherein the solution contains a by-products of oxidation of diacetone alcohols with hypochlorous acid or salt thereof and/or 2,3-dihydroxy-3-methylbutanoic acid and 3-hydroxy-3-methylbutyrate and impurities in the solution are exposed to environments of pH6.0 or less, and the treatment step is characterized to perform at least one of the following operations (a) to (d) on the solution.

(a) Hypochlorous acid or salt thereof is added to the solution.
(b) The solution is maintained at 40 to 200°C for 30 minutes or more, provided that the solution contains hypochlorous acid or salt thereof.
(c) Calcium salt is added to the solution, and the precipitated calcium 2,3-dihydroxy-3-methylbutanoate is separated from the liquid phase in which 3-hydroxy-3-methylbutyrate and/or salt thereof is dissolved.
(d) A base is added to the solution to separate the liquid phase in which the salt of 2,3-dihydroxy-3-methylbutanoic acid formed is dissolved and the liquid phase in which 3-hydroxy-3-methylbutyrate and/or salt thereof is dissolved.

[Formula 1]

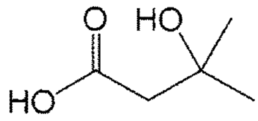

3-hydroxy-3-methylbutyrate
(HMB)

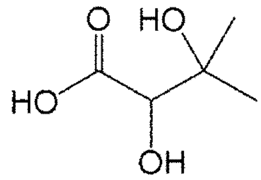

2,3-dihydroxy-3-methylbutanoic acid
(DHMB)

(2) The method for producing 3-hydroxy-3-methylbutyrate or salt thereof according to (1), wherein the preparation step comprises reacting diacetone alcohol with hypochlorous acid (HClO) or salt thereof (MClO) to form 3-hydroxy-3-methylbutyrate.
[Formula 2]

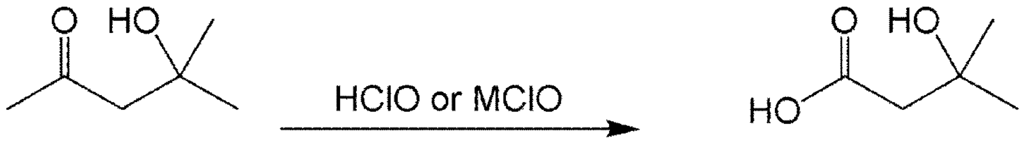


diacetone alcohol
(DAA)

3-hydroxy-3-methylbutyrate
(HMB)

(3) The method for producing 3-hydroxy-3-methylbutyrate or salt thereof according to (1) or (2), wherein the content of 3-hydroxy-3-methylbutyrate in the solution is 0.01 to 10 mol/1.
(4) The method for producing 3-hydroxy-3-methylbutyrate or salt thereof according to any one of (1) to (3), wherein the amount of added hypochlorous acid or salt thereof in the operation of (a) is 0.0005 to 10 times as the amount of substance converted to the content of 3-hydroxy-3-methylbutyrate in the solution.
(5) The method for producing 3-hydroxy-3-methylbutyrate or salt thereof according to any one of (1) to (4), wherein the amount of added calcium salt in the operation (c) is 0.1 to 10 times as the amount of substance converted to the content of 2,3-dihydroxy-3-methylbutanoic acid in the solution.
(6) The method for producing 3-hydroxy-3-methylbutyrate or salt thereof according to any one of (1) to (5), wherein the amount of added base in the operation (d) is 0.1 to 10 times as the amount of substance converted to the content of 2,3-dihydroxy-3-methylbutanoic acid in the solution.
(7) The method for producing 3-hydroxy-3-methylbutyrate or salt thereof according to any one of (1) to (6), further comprising a separation step of adding calcium hydroxide to the solution obtained through the treatment step and separating the precipitated calcium 3-hydroxy-3-methylbutanoate from the liquid phase.

EFFECT OF INVENTION

According to the present invention, it is possible to efficiently produce highly pure 3-hydroxy-3-methylbutyrate or salt thereof.

BRIEF DESCRIPTION OF THE DRAWINGS

Fig. 1 shows the results of high-performance liquid-chromatography (HPLC) measurements of the reaction mixtures after the reaction in the reference examples.
Fig. 2 shows the results of high performance liquid chromatography (HPLC) measurements of HMBs of free acids extracted and recovered in reference examples.
Fig. 3 shows the results of high performance liquid chromatography (HPLC) measurements of recovered calcium salts of HMBs in the reference examples.

DETAILED DESCRIPTION OF THE INVENTION

In explaining the details of the present invention, a specific example will be described, and the present invention is not limited to the following contents as long as it does not deviate from the gist of the present invention, and can be implemented by appropriately changing.

<The method for producing 3-hydroxy-3-methylbutyrate or salt thereof>

The method for producing 3-hydroxy-3-methylbutyrate or salt thereof, which is one embodiment of the present invention (hereinafter, sometimes abbreviated as "the production method of the present invention"), is a method including a "preparation step for preparing a solution containing 3-hydroxy-3-methylbutyrate and impurities (hereinafter, sometimes abbreviated as "the preparation step")" and a "treatment step for reducing or eliminating impurities in the solution (hereinafter, sometimes abbreviated as "the treatment step")". The solution contains, as impurities, by-products of the oxidation reaction of diacetone alcohol by hypochlorous acid or salt thereof and/or 2,3-dihydroxy-3-methylbutanoic acid, 3-hydroxy-3-methylbutyrate and impurities in the solution are exposed to environments of pH 6.0 or less, and the treatment step is a step of performing at least one of the following operations (a) to (d) on the solution.

(a) Hypochlorous acid or salt thereof is added to the solution.
(b) The solution is maintained at 40 to 200°C for 30 minutes or more, provided that the solution contains hypochlorous acid or salt thereof.
(c) Calcium salt is added to the solution, and the precipitated calcium 2,3-dihydroxy-3-methylbutanoate is separated from the liquid phase in which 3-hydroxy-3-methylbutyrate and/or the salt is dissolved.
(d) A base is added to the solution to separate the liquid phase in which the salt of 2,3-dihydroxy-3-methylbutanoic acid formed is dissolved and the liquid phase in which 3-hydroxy-3-methylbutyrate and/or salt thereof is dissolved.

[Formula 3]

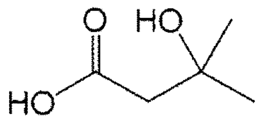

3-hydroxy-3-methylbutyrate (HMB)

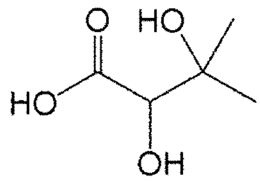

2,3-dihydroxy-3-methylbutanoic acid (DHMB)

As a typical method for producing 3-hydroxy-3-methylbutyrate, there is a widely known method using the oxidation reaction of diacetone alcohol with hypochlorite, but if this reaction is simply used, there is a problem that impurities such as reaction intermediates and by-products of 2,3-dihydroxy-3-methylbutanoic acid are mixed in, and a technique for removing this is necessary. For example, as described in Patent Document 2, a method of purifying by dissolving the obtained salt, acidifying the salt, and then extracting the salt may be considered, but such a method complicates the process, cannot remove impurities sufficiently, and it has been difficult to efficiently produce highly pure 3-hydroxy-3-methylbutyrate or salt thereof.

The inventors have found that highly pure 3-hydroxy-3-methylbutyrate or salt thereof can be efficiently produced by exposing 3-hydroxy-3-methylbutyrate and impurities to environments of pH 6.0 or less and performing at least one of the operations (a) to (d). The operations in (a) and (b) are particularly intended to reduce or eliminate "by-products of the oxidation of diacetone alcohol by hypochlorous acid or salt thereof" and it is considered that "hypochlorous acid or salt thereof" plays a role for the decomposition of the by-products exposed to environments of pH 6.0 or less to 3-hydroxy-3-methylbutyrate or 2,3-dihydroxy-3-methylbutanoic acid and others. On the other hand, the operations in (c) and (d) are operations for reducing or eliminating "2,3-dihydroxy-3-methylbutanoic acid" in particular, and the operations in (c) are for preferentially precipitating the calcium salt of 2,3-dihydroxy-3-methylbutanoic acid by addition of the calcium salt, so that 2,3-dihydroxy-3-methylbutanoic acid and 3-hydroxy-3-methylbutyrate can be easily separated.

In the operation (d), the salt of 2,3-dihydroxy-3-methylbutanoic acid is preferentially dissolved in a water-soluble liquid by addition of a base, so that the aqueous phase in which dissolved 2,3-dihydroxy-3-methylbutanoic acid and the liquid phase in which dissolved 3-hydroxy-3-methylbutyrate can be easily separated by liquid-liquid extraction.

The producing method of the present invention can efficiently produce highly pure 3-hydroxy-3-methylbutyrate

or salt thereof by a relatively simple method, and can be said to be an industrially excellent method.

Hereinafter, "the preparation step", "the treatment step", and others will be described in detail.

The preparation step

The preparation step is a step of preparing a solution containing 3-hydroxy-3-methylbutyrate (hereinafter, sometimes abbreviated as "HMB") and impurities (hereinafter, sometimes abbreviated as "solution"), and the solution contains by-products of the oxidization of diacetone alcohol by hypochlorous acid or salt thereof and/or 2,3-dihydroxy-3-methylbutanoic acid (hereinafter, sometimes abbreviated as "DHMB") as impurities, and the solution containing 3-hydroxy-3-methylbutyrate and impurities may be treated as a liquid at room temperature, and therefore, the solution may be a solution in which impurities are dissolved in a non-solvent, for example, HMB, since HMB is a liquid at room temperature.

In addition, "3-hydroxy-3-methylbutyrate and impurities in the solution" are exposed to environments of pH 6.0 or less, then as long as the HMB and the impurities are exposed to pH 6.0 or less once, and there is no particular limitation on the method of preparing the solution from the HMB and the impurities which are exposed to environments of pH 6.0 or less. For example, the solution in which HMB and impurities are dissolved may be set to pH 6.0 or less, or may be an extract obtained by concentrating a solution in which pH 6.0 or less is set, or an extract or a concentrate thereof obtained by liquid-liquid extracting HMB and impurities from a solution in which pH 6.0 or less is set.

The solution may be solvent-free, as described above, and preferably comprises a solvent. Examples of the solvent include water; alcohols such as ethanol, i-propanol, ethylene glycol and glycerin; hydrocarbon solvents such as hexane and heptane; ether solvents such as tetrahydrofuran (THF) and 1,2-dioxane; ester solvents such as ethyl acetate; halogen solvents such as methylene chloride and chloroform; and aprotic polar solvents such as acetone and dimethyl sulfoxide (DMSO), and others, and the solvent may be a mixed solvent of two or more solvents. Among them, water and ethyl acetate are particularly preferable.

The content of HMB in the solution is not particularly limited, and can be appropriately selected depending on the purpose, and is usually 0.01 to 10mol/l, preferably 0.4mol/l or more, more preferably 1.4mol/l or more, preferably 9.5mol/l or less, more preferably 9.3mol/l or less.

When the content of HMB in the solution is within the above range, HMB or salt thereof can be easily produced more efficiently.

The content of the "by-products of the oxidation reaction of diacetone alcohol by hypochlorous acid or salt thereof" in the solution is not particularly limited, and is usually 0.000001 to 5 times, preferably 0.00001 times or more, more preferably 0.00005 times or more, and 1 times or less, more preferably 0.5 times or less as the amount of substance converted to the content of HMB

The content of DHMB in the solution is not particularly limited, and the solution is usually 0.001 to 5 times, preferably 0.005 times or more, more preferably 0.01 times or more, preferably 1 times or less, more preferably 0.5 times or less as the amount of substance converted to the content of HMB

The pH (hydrogen ion concentration) to which the HMB and impurities are exposed in the solution is 6.0 or less, preferably 5.0 or less, more preferably 4.0 or less, more preferably 3.0 or less, particularly preferably 2.0 or less, most preferably 1.0 or less, usually 0.5 or more.

When the pH (hydrogen ion concentration) to which the HMB and impurities are exposed in the solution is within the above range, HMB or salt thereof can be easily produced more efficiently.

The preparation method of the solution in the preparation step is not particularly limited, and the solution itself may be obtained, the solution may be prepared by obtaining HMB containing DHMB and others or the solution may be prepared by synthesizing HMB containing DHMB and others. Hereinafter, a process for synthesizing HMBs containing DHMB and others to prepare solutions will be described in detail.

As a method for synthesizing HMB which contains DHMB and others, there is a method for producing HMB by reacting diacetone alcohol (hereinafter, sometimes abbreviated as "DAA") with hypochlorous acid (HClO) or salt thereof (MClO) (hereinafter, sometimes abbreviated as "DAA oxidizing reaction").

[Formula 4]

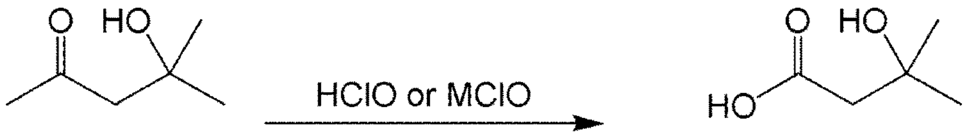


diacetone alcohol (DAA)

3-hydroxy-3-methylbutyrate (HMB)

Such a process is the so-called "oxidation reaction of diacetone alcohol with hypochlorous acid or salt thereof", which produces "DHMB" and others "by-products of the oxidation reaction of diacetone alcohol with hypochlorous acid or salt thereof". Therefore, it is preferable to utilize the producing method of the present invention.

Hypochlorous acid (HClO) or salt thereof (MClO) includes hypochlorous acid, sodium hypochlorite, and calcium hypochlorite, and sodium hypochlorite is particularly preferred.

The amount of hypochlorous acid (HClO) or salt thereof (MClO) used in the DAA oxidization reaction is usually 3 to 5 times, preferably 3.2 times or more, more preferably 3.4 times or more, preferably 4 times or less, more preferably 3.8 times or less as the amount of substance converted to the content of DAA.

When the amount of hypochlorous acid (HClO) or salt thereof (MClO) used in the DAA oxidization reaction is within the above range, HMB or salt thereof can be easily produced more efficiently.

Examples of the solvent for the DAA oxidation reaction include water, a mixed solvent of an alcohol such as an aqueous solution of ethanol and water, and a mixed solvent of an ester solvent such as ethyl acetate and water.

The reaction temperature of the DAA oxidation reaction is usually -15 to 60°C, preferably -5 °C or higher, more preferably 0 °C or higher, preferably 20°C or less, more preferably 10°C or less.

The reaction time of the DAA oxidation reaction is usually 5 minutes to 24 hours, preferably 10 minutes or more, more preferably 30 minutes or more, preferably 8 hours or less, more preferably 2 hours or less.

When the reaction temperature or the reaction time of the DAA oxidation reaction is within the above range, HMB or salt thereof can be easily produced more efficiently.

The method of preparing the solution after completing the DAA oxidation reaction includes any one of the following (I) to (VI).

(I) A method in which a reducing agent is added to a reaction solution or reaction solution of a DAA oxidation reaction, and an acid or an acid and a base are added to a deactivating solution obtained by deactivating some or all of hypochlorous acid or salt thereof used in excess (hereinafter, the reaction solution or deactivating solution may be abbreviated as a "reaction mixture") to adjust the pH to 6.0 or less.

(II) A method for concentrating the solution after adding an acid or an acid and a base to the reaction mixture to adjust the pH to 6.0 or less.

(III) A method for extracting HMB and impurities by liquid-liquid extraction after adding an acid or an acid and a base to the reaction mixture to adjust the pH to 6.0 or less.

(IV) A method for extracting HMB and impurities by liquid-liquid extraction after adding an acid or an acid and a base to the reaction mixture to adjust the pH to 6.0 or less, followed by a concentration of the solution.

(V) A method in which an acid or an acid and a base are added to the reaction mixture to adjust the pH to 6.0 or less, followed by liquid-liquid extraction to extract HMB and impurities, and then the extract is concentrated.

(VI) A method for extracting HMB and impurities by liquid-liquid extraction after adding an acid or an acid and a base to the reaction mixture to adjust the pH to 6.0 or less, followed by a concentration of the solution, and then the extract is concentrated.

The type of reducing agent for deactivating a part or all of the hypochlorous acid or salt thereof used in excess is not particularly limited, and a known reducing agent can be appropriately used, and thiosulfate, sulfite and others are preferable, and sodium thiosulfate is particularly preferable. As a method for deactivating a part or all of the hypochlorous acid or salt thereof used in excess, a method of decomposing the hypochlorous acid or salt thereof at a pH of 6.0 or less may be used instead of using a reducing agent.

The type of the acid and base for setting the pH to 6.0 or less is not particularly limited, and well-known organic acids, inorganic acids, organic bases, inorganic bases, and others can be used as appropriate, and inorganic acids such as hydrochloric acid (HCl), sulfuric acid ($H_2SO$), nitric acid ($HNO_3$), and others, inorganic bases such as sodium hydroxide (NaOH) are preferable, and hydrochloric acid and sodium hydroxide are particularly preferable.

The treatment step

The treatment step is a step of "reducing or eliminating impurities in the solution prepared in the preparation

step" and performs at least one of the following operations (a) to (d) on the solution, and the operations (a) to (d) are not limited to only one type of operation, and two or more types of operations may be combined. Among these, a combination of the operations of (a) and (c) and a combination of the operations of (a) and (d) are preferable.

(a) Hypochlorous acid or salt thereof is added to the solution.
(b) The solution is maintained at 40 to 200°C for 30 minutes or more, provided that the solution contains hypochlorous acid or salt thereof.
(c) Calcium salt is added to the solution, and the precipitated calcium 2,3-dihydroxy-3-methylbutanoate is separated from the liquid phase in which 3-hydroxy-3-methylbutyrate and/or the salt is dissolved.
(d) Abase is added to the solution to separate the liquid phase in which the salt of 2,3-dihydroxy-3-methylbutanoic acid formed is dissolved and the liquid phase in which 3-hydroxy-3-methylbutyrate and/or salt thereof is dissolved.

Hereinafter, the operations of (a) to (d) will be described in detail.

The operation (a) is an operation of adding hypochlorous acid or salt thereof to a solution, and hypochlorous acid or salt thereof includes hypochlorous acid, sodium hypochlorite, and calcium hypochlorite, and sodium hypochlorite is preferable, and an aqueous solution of sodium hypochlorite is particularly preferable.

The amount of hypochlorous acid or salt thereof added in the operation (a) is usually 0.0005 to 10 times, preferably 0.005 times or more, more preferably 0.01 times or more, preferably 1 times or less, more preferably 0.5 times or less, as the amount of substance converted to the content of HMB in the solution.

When the amount of hypochlorous acid or salt thereof added is within the above range, HMB or salt thereof can be easily produced more efficiently.

The operation (b) is an operation of holding the solution at 40 to 200°C for 30 minutes or more.

The holding temperature of the solution is preferably 100°C or less, more preferably 80°C or less, preferably 50 °C or more, more preferably 60°C or more. The retention time of the solution is 30 minutes or more, preferably 2 hours or more, more preferably 4 hours or more, and usually 12 hours or more.

When the holding temperature and the holding temperature of the solution are within the above range, HMB or salt thereof can be more efficiently produced.

The solution in the operation (b) is one containing hypochlorous acid or salt thereof, and the content of hypochlorous acid or salt thereof is usually 0.0005 to 10 times, preferably 0.005 times or more, more preferably 0.01 times or more, preferably 1 times or less, more preferably 0.5 times or less as the amount of substance converted to the content of HMB When the amount of hypochlorous acid or salt thereof added is within the above range, HMB or salt thereof can be easily produced more efficiently.

The operation (c) is an operation in which calcium salt is added to the solution and calcium 2,3-dihydroxy-3-methylbutanoate preferentially precipitated is separated from the liquid phase in which 3-hydroxy-3-methylbutyrate and/or salt thereof is dissolved, and the type of anion of the calcium salt is not particularly limited, and specifically, hydroxide ion, carbonate ion, and others.

Examples of calcium salts include calcium hydroxide, calcium carbonate, calcium bicarbonate, and others.

Also, "precipitated calcium 2,3-dihydroxy-3-methylbutanoate" is not limited to calcium DHMB alone, and may be complex salts such as hydrates, solvates, and DHMB-Ca-HMB of calcium DHMB.

As a method of adding the calcium salt, a method of directly adding the calcium salt in a state of powder and others without dissolving it in a solvent and others is preferable.

The amount of the calcium salt added in the operation (c) is usually 0.1 to 10 times, preferably 0.2 times or more, more preferably 0.25 times or more, preferably 2 times or less, more preferably 1 times or less as the amount of substance converted to the content of DHMB in the solution.

When the amount of the calcium salt added is within the above range, HMB or the salt thereof can be easily produced more efficiently.

The temperature for precipitating calcium DHMB in the operation of (c) is usually -15 to 40°C, preferably 30 °C or less, more preferably 10°C or less, more preferably -10°C or more, more preferably -5°C or more.

When the temperatures for precipitating calcium DHMB is within the above range, HMBs or salt thereof can be more efficiently produced.

The operation of separating the precipitated calcium 2,3-dihydroxy-3-methylbutanoate from the liquid phase in which 3-hydroxy-3-methylbutyrate and/or salt thereof are dissolved is an operation of separating solid and liquid by filtration or decantation.

The operation (d) is an operation in which a base is added to the solution to separate the liquid phase in which a salt of 2,3-dihydroxy-3-methylbutanoic acid formed is preferentially dissolved and the liquid phase in which 3-hydroxy-3-methylbutyrate and/or salt thereof is dissolves.

The type of the base is not particularly limited, and a known organic base, an inorganic base and others can be used as appropriate, and concretely, an inorganic base such as a hydroxide of an alkali metal or a hydroxide of an

alkaline earth metal and others. Examples of the alkali metal hydroxide include sodium hydroxide and the alkaline earth metal hydroxide includes calcium hydroxide.

Examples of the method of adding the base include a method of directly adding the base in the form of a powder and others, and a method of directly adding the base in the form of an aqueous solution or a water-soluble solvent, and when the base is directly added in the form of a powder and others, it is necessary to previously contain water or a water-soluble solvent, or thereafter add water or a water-soluble solvent.

The amount of the base added in the operation (d) is usually 0.1 to 10 times, preferably 0.2 times or more, more preferably 0.25 times or more, preferably 2 times or less, more preferably 1.5 times or less as the amount of substance converted to the content of DHMB in the solution.

When the amount of the base added is within the above range, HMB or salt thereof can be easily produced more efficiently.

The temperature for separating the liquid phase in which DHMB salts dissolve and the liquid phase in which HMBs dissolve in the operation (d) is usually -5 to 90 °C, preferably 70 °C or less, more preferably 60 °C or less, and preferably 0 °C or more. When the temperature for separating the liquid phase is within the above range, HMB or salt thereof can be easily produced more efficiently.

The liquid phase in which the salts of DHMB are dissolved in the process (d) is a phase composed of water, methanol, ethanol, and others water-soluble solvents. The liquid phase in which the HMB and/or the salt thereof dissolves in the operation (d) is a phase consisting of a non-aqueous solvent such as ethyl acetate or a poorly water-soluble solvent.

Separation of the liquid phase in which the salt of 2,3-dihydroxy-3-methylbutanoic acid dissolves and the liquid phase in which the salt of 3-hydroxy-3-methylbutyrate and/or salt thereof dissolves is carried out by separation of liquids and liquids.

The production method of the present invention is not particularly limited as long as it includes the above preparation step and treatment step, and it is preferable to further include a separation step of adding calcium hydroxide to the solution obtained through the treatment step and separating the precipitated calcium 3-hydroxy-3-methylbutanoate from the liquid phase. Note that the "precipitated calcium 3-hydroxy-3-methylbutanoate" is not limited to HMB calcium alone, and may be hydrates, solvates and others of HMB calcium.

The addition amount of calcium hydroxide in the separation step is usually 0.1 to 2 times, preferably 0.4 times or more, more preferably 0.45 times or more, preferably 0.6 times or less, more preferably 0.55 times or less as the amount of substance converted to the content of HMB in the solution.

When the amount of added calcium hydroxide is within the above range, HMB or salt thereof can be easily produced more efficiently.

The temperature for precipitating the HMB calcium in the separation step is usually -15 to 50°C, preferably 30 °C or less, more preferably 10°C or less, more preferably -10°C or more, more preferably -5°C or more.

When the temperature for precipitating HMB calcium is within the above range, HMB or salt thereof can be easily produced more efficiently.

## EXPERIMENTALS

### <Reference Example>

The flasks were charged with 347 g (0.620 mol) of an aqueous solution of sodium hypochlorite at a consistency of 13.3 wt%, cooled, and 20 0 g (0.172 mol) of diacetone alcohol was added while maintaining at 0 to 10°C, and stirred at 0 to 10 °C for 15 minutes to react the diacetone alcohol and sodium hypochlorite. The reaction mixture after the reaction was subjected to high performance liquid chromatography (HPLC) under the conditions shown in Table 1 below. The chart of the measurement results is shown in FIG. 1, and the peak areas are shown in Table 2.

The reaction mixture was then acidified (pH 1.0) with 60.8 g of hydrochloric acid (0.600 mol), concentrated to a residue weight of 125 g (about 30 % of the pre-concentration weight), the free acid 3-hydroxy-3-methylbutyrate (HMB) was extracted and recovered with ethyl acetate (45 ml×3 times), and the combined three extracts were concentrated to give a HMB14.9 g of the free acid (0.126 mol in terms of HMB). The HMBs of the free acids extracted and recovered were similarly measured for HPLC. The chart of the measurement results is shown in FIG. 2 and the peak areas are shown in Table 2. Further, the obtained HMB 14.7g (0.124 mol in terms of HMB) was charged into flasks, and 85 ml of a 95 vol. % aqueous solution of ethanol was added to the flasks to dissolve them, and 4.77 g (0.064 mol) of calcium hydroxide was added to this solution to neutralize (pH 7.6), and the flasks were cooled to 0 to 5°C. The precipitated crystals were filtered and dried to collect 17. 8 g of HMB-calcium salts. The HPLC of the collected calcium salt of HMB was also measured. The chart of the measurement results is shown in FIG. 3, and the peak areas are shown in Table 2.

Table 1

Table 1 Testing condition

| | |
|---|---|
| Detector | An ultraviolet absorption photometer (measurement wavelength : 210mn) |
| Column | A stainless steel column 4.6 mm in inside diameter and 15 cm in length, packed with octadecylsilanized silica gel with a particle size of 5 μm for liquid chromatography. |
| Column temperature | A constant temperature of about 35°C |
| Mobile phase | 50 mM ammonium dihycrogen phosphate reagent (note 1) in acetonitrile (95:5 by volume) |
| Flow rate | 1.0 ml/min |
| Note 1: 5.75 g of ammonium dihydrogen phosphate was dissolved in water to make 1000 L. Phosphoric acid was added to this solution to adjust the pH to 3.0. | |

Table 2

Table 2

| | DHMB area % (vs. HMB) | By-products area % (vs. HMB) | HMB area % (vs. total area) |
|---|---|---|---|
| After reaction | 16.0 | 34.1 | 53.3 |
| Extraction and recovered HMB of the free acid | 15.7 | 9.8 | 75.1 |
| Recovered calcium salt of HMB | 7.3 | 2.6 | 89.8 |

DHMB area % (vs. HMB) = peak area of DHMB/(peak area of DHMB + peak area of HMB) × 100

By-products area % (vs. HMB)
= peak area of by-products/(peak area of by-products + peak area of HMB) × 100

HMB area % (vs. total area) = peak area of HMB/(sum of areas of detected peaks) × 100

<Example 1> (Operation b)

The flasks were charged with 694 g (1.24 mol) of an aqueous solution of 13.3 wt% sodium hypochlorite, cooled, and 40.0 g (0.344 mol) of diacetone alcohol was added while maintaining at 0 to 10 °C, the stirring was continued for 30 minutes at 0 to 10 °C to react the diacetone alcohol with sodium hypochlorite, and the reaction mixture was acidified with hydrochloric acid to the pH shown in Table 3 below, respectively (it is estimated that hypochlorous acid and/or sodium hypochlorite were dissolved 0.1 times as the amount of substance converted to the content of DAA used in the acidified solution). The solutions were stored at 60 °C for 6 hours, and the quantity of by-products after storage (by-products of the oxidative reactions of DAA by NaClO) was calculated by HPLC measurements according to the condition of Table 1. The results are indicated in the following table 3.

Table 3

Table 3

| | By-products area % (vs. HMB) |
|---|---|
| After reaction (pH14) | 32.9 |
| No acidification (pH14) and stored | 30.7 |
| Acidified to pH10 and stored | 33.0 |
| Acidified to pH8 and stored | 31.4 |
| Acidified to pH6 and stored | 27.1 |
| Acidified to pH4 and stored | 13.1 |
| Acidified to pH2 and stored | 9.6 |
| Acidified to pH1 and stored | 0.4 |

<Example 2> (Operation a)

The flasks were charged with 694 g (1.24 mol) of an aqueous solution of 13.3 wt% sodium hypochlorite, cooled, and 40.0 g (0.344 mol) of diacetone alcohol was added while maintaining at 0 to 10 °C, the stirring was continued for 30 minutes at 0 to 10 °C to react the diacetone alcohol with sodium hypochlorite, and the reaction mixture was acidified with hydrochloric acid to the pH indicated in Table 4 below. This solution was added an aqueous solution of sodium hypochlorite having a concentration of 13.3 wt% (0.5 times in terms of the amount of the DAA used) and stirred at 30 °C. The quantity of by-products after 1 hour of addition (by-products of the oxidative reactions of DAA by NaClO) was calculated by HPLC measurements according to the condition of Table 1. The results are indicated in the following table 4.

Table 4

Table 4

| | Reaction intermediates area % (vs. HMB) |
|---|---|
| After reaction (pH14) | 32.9 |
| No acidification (pH14), sodium hypochlorite added for 1 hour | 32.1 |
| Acidify to pH10, 1h after addition of sodium hypochlorite | 33.0 |
| Acidify to pH8, 1h after addition of sodium hypochlorite | 34.0 |
| Acidify to pH6, 1h after addition of sodium hypochlorite | 23.2 |
| Acidify to pH2. 1h after addition of sodium hypochlorite | 19.3 |
| Acidify to pH1, 1h after addition of sodium hypochlorite | 2.6 |

<Example 3> (Operation a)

HMB 100 g containing by-products (by-products of the oxidation of DAA by NaClO), 25 g of water and an aqueous solution of sodium hypochlorite having a strength of 12.6 wt% were added in the amounts shown in Table 5 below and stirred at 30 °C for 30 minutes to 2 hours. The quantity of by-products after the addition/stirring (by-products of the oxidative reactions of DAA by NaClO) was calculated by HPLC measurements according to the condition of Table 1. The results are indicated in the following table 5.

Table 5

Table 5

| | By-products area % (vs. HMB) |
|---|---|
| HMB containing by-products | 12.3 |
| Addition of 0.005 times sodium hypochlorite (as the amount of substance converted to the content of HMB) | 8.5 |
| Addition of 0.01 times sodium hypochlorite (as the amount of substance converted to the content of HMB) | 4.1 |
| Addition of 0.015 times sodium hypochlorite (as th e amount of substance converted to the content of HMB) | 0.1 |
| Addition of 0.025 times sodium hypochlorite (as the amount of substance converted to the content of HMB) | 0.2 |
| Addition of 0.04 times sodium hypochlorite (as the amount of substance converted to the content of HMB) | 0.2 |

<Example 4> (Operation a)

HMB 15.0 g containing a by-products (by-products of the oxidative reactions of DAA by NaClO) was added 2.9 g and 60 ml of ethyl acetate to make a solution to which was added an aqueous solution of sodium hypochlorite at a concentration of 13.3 wt% in the amount shown in Table 6 below and stirred at 30 °C for 1 hour. The quantity of by-products after the addition/stirring (by-products of the oxidative reactions of DAA by NaClO) was calculated by HPLC measurements according to the condition of Table 1. The results are indicated in the following table 6.

Table 6

Table 6

| | By-products area % (vs. HMB) |
|---|---|
| HMB containing by-products (ethyl acetate solution) | 9.6 |
| Addition of 0:04 times sodium hypochlorite (as the amount of substance converted to the content of HMB) | 0.0 |

<Example 5> (Operation a and b)

The flasks were charged with 311 g (0.543 mol) of an aqueous solution of sodium hypochlorite having a concentration of 13.0 wt%, cooled, 18.0 g (0.155 mol) of diacetone alcohol was added while maintaining at 0 to 10 °C, the stirring was continued for 30 minutes at 0 to 10 °C to react the diacetone alcohol with sodium hypochlorite, the reaction mixture was acidified (pH 0.9) with 55.0 g (0.543 mol) of hydrochloric acid, the reaction mixture was concentrated (concentrated under reduced pressure while maintaining at 50 to 60 °C for 3 hours) until the residue weight was 70 g (about 20 % of the weight before concentration), the HMB of free acid was extracted and recovered with 73 ml of ethyl acetate, and the aqueous solution of sodium hypochlorite having a concentration of 13.0 wt% was added to the extracted solution, and the reaction mixture was stirred at 33 °C for 3 hours as described in Table 7. The amounts of by-products (by-products of the oxidative reactions of DAA by NaClO) in each instance were calculated by HPLC measurements according to the condition of Table 1. The results are indicated in the following table 7.

Table 7

Table 7

| | By-products area % (vs. HMB) |
|---|---|
| After reaction | 33.6 |
| Extracted and recovered HMB with ethyl acetate (Extracted and recovered solution) | 5.3 |
| Addition of 0.025 times sodium hypochlorite (as the amount of substance converted to the content of HMB) | 0.0 |

<Example 6> (Operation c)

The flasks were charged with 311 g (0.543 mol) of an aqueous solution of sodium hypochlorite having a concentration of 13.0 wt%, cooled, and 18.0 g (0.155 mol) of diacetone alcohol was added while maintaining at 0 to 10 °C, the stirring was continued for 30 minutes at 0 to 10 °C to react the diacetone alcohol with sodium hypochlorite, the reaction mixture was acidified (pH 0.8) with 56.0 g (0.553 mol) of hydrochloric acid, the reaction mixture was concentrated to a residue weight of 95 g (about 30 % of the weight before concentration), the HMB of the free acid was extracted and recovered with ethyl acetate (20 ml×4 times), and the four extracts were combined. This combined solution, calcium hydroxide (0.45 times as the amount of substance converted to the content of 2,3-dihydroxy-3-methylbutanoic acid (DHMB)) was added, and the DHMB calcium salt was cooled to 0 to 5 °C and precipitated. This was separated by filtration, and the quantity of DHMB after filtration was calculated by HPLC measurements according to the condition of Table 1. The results are indicated in the following table 8.

Table 8

Table 8

| | DHMB area % (vs. HMB) |
|---|---|
| Extracted and recovered HMB with ethyl acetate (Extracted and recovered solution) | 33.8 |
| Addition of calcium hydroxide 0.45 times (as the amount of substance converted to the content of DHMB), filtration of precipitates | 0.4 |

<Example 7> (Operation of c)

HMB 8.3g containing 2,3-dihydroxy-3-methylbutanoic acid (DHMB), 2.5 g of water and 33 ml of ethyl acetate were added to dissolve, calcium hydroxide was added to the solution in the amounts shown in Table 9 below to precipitate DHMB calcium salts, which were separated by filtration at 0 to 5 °C, and the amount of DHMB after filtration was calculated by HPLC measurements according to the condition of Table 1. The results are indicated in the following table 9.

Table 9

Table 9

| | DHMB area % (vs. HMB) |
|---|---|
| HMB containing DHMB | 9.7 |
| Addition of 0.13 times calcium hydroxide (as the amount of substance converted to the content of DHMB), filtration of precipitates | 4.6 |
| Addition of 0.24 times calcium hydroxide (as the amount of substance converted to the content of DHMB), filtration of precipitates | 1.0 |
| Addition of 0.29 times calcium hydroxide (as the amount of substance converted to the content of DHMB), filtration of precipitates | 0.5 |

(continued)

| | DHMB area % (vs. HMB) |
|---|---|
| Addition of 0.40 times calcium hydroxide (as the amount of substance converted to th e content of DHMB), filtration of precipitates | 0.5 |
| Addition of 0.50 times calcium hydroxide (as the amount of substance converted to the content of DHMB), filtration of precipitates | 0.6 |
| Addition of 0.66 times calcium hydroxide (as the amount of substance converted to the content of DHMB), filtration of precipitates. | 0.6 |

<Example 8> (Operation a, b and c)

The flasks were charged with 311 g (0.543 mol) of an aqueous solution of sodium hypochlorite having a concentration of 13.0 wt%, cooled, 18.0 g (0.155 mol) of diacetone alcohol was added while maintaining at 0 to 10 °C, the stirring was continued for 30 minutes at 0 to 10 °C to react the diacetone alcohol with sodium hypochlorite, the reaction mixture was acidified (pH 0.9) with 55.6g (0.549 mol) of hydrochloric acid, the reaction mixture was concentrated (concentrated under reduced pressure while maintaining at 50 to 60 °C for 3 hours) until the residue weight was 101 g (about 30% of the weight before concentration), the HMB of the free acid was extracted and recovered with ethyl acetate (30 ml×1 time, 20 ml×3 times), and the four extracts were combined. This combined solution was added an aqueous solution of sodium hypochlorite having a concentration of 13.0 wt% (0.02 times as the amount of substance converted to the content of DAA used) and stirred at 30 to 32 °C for 3 hours. Subsequently, 0.25 g of hydrochloric acid was added to this solution to acidify the solution to pH 0.4, and then calcium hydroxide (0.45-fold mol as the contained 2,3-dihydroxy-3-methylbutanoic acid (DHMB)) was added to cool the DHMB calcium salt at 0 to 5 °C to precipitate, and this was separated by filtration. Subsequently, 3 ml of water was added to the filtrate, HMBs of free acids were extracted and recovered, and then concentrated to obtain highly pure HMB 10.2 g (0.086 mol). The obtained HMBs were charged into flasks, dissolved by adding 59 ml of a 95 volume % aqueous solution of ethanol, and neutralized (pH 7. 8) by adding 3.16 g (0.043 mol) of calcium hydroxide in the solution, and cooled to 0 to 5 °C. The precipitated crystals were filtered and dried to collect 10.0 g of highly pure HMB-calcium salts. The amounts of by-products (by-products of the oxidative reactions of DAA by NaClO) and the amounts of DHMB and others in the respective cases were calculated by HPLC measurements according to the condition of Table 1. The results are indicated in the following table 10.

Table 10

Table 10

| | DHMB area % (vs. HMB) | By-products area % (vs. HMB) | HMB area % (vs. total area) |
|---|---|---|---|
| After reaction | 18.8 | 34.1 | 44.6 |
| Extracted and recovered HMB with ethyl acetate (Extracted and recovered solution) | 19.7 | 11.3 | 70.4 |
| After addition of sodium hypochlorite | 20.3 | 0. 0 | 76.3 |
| After addition of calcium hydroxide and filtration of precipitates (highly pure HMB) | 0.2 | 0.0 | 98.0 |
| Highly pure calcium salt of HMB | 0.1 | 0.0 | 99.8 |

<Example 9> (Operation a and c)

The reaction kettle was charged with 601.1 kg (5.09 kmol) of HMB and 60 l of water, which contained by-products (by-products of the oxidation reaction of DAA by NaClO) and 2,3-dihydroxy-3-methylbutanoic acid (DHMB), dissolved, and 98 kg (0.174 kmol) of an aqueous solution of sodium hypochlorite at a consistency of 13.2 % was added and stirred for 3 hours at 27 to 32 °C. After 12.8 kg (0.126 kmol) of hydrochloric acid was added to acidify the pH to 0.7, calcium hydroxide (0.45 times as the amount of substance converted to the content of 2,3-dihydroxy-3-methylbutanoic

acid (DHMB)) and 2405 l of ethyl acetate were added, and then the mixture was cooled to -5 to 5 °C to precipitate a DHMB calcium salt, which was then separated by filtration. After extracting and recovering the HMB of the free acid, the HMB was concentrated to obtain 4.22 kmol of highly pure HMB 498.5kg. The HMB thus obtained was charged into a reaction kettle, 2955 1 of a 95 vol. % aqueous solution of ethanol was added to dissolve the HMB, 149.2 kg (2.01 kmol) of calcium hydroxide was added to the solution, neutralized (pH 8.0), and cooled to -5 to 10 °C. The precipitated crystals were filtered and dried to collect 502.5 kg of highly pure calcium salt of HMB. The amounts of by-products (by-products of the oxidative reactions of DAA by NaClO) and the amounts of DHMB and others in the respective cases were calculated by HPLC measurements according to the condition of Table 1. The results are indicated in the following table 11.

Table 11

Table 11

| | DHMB area % (vs. HMB) | By-products area % (vs. HMB) | HMB area % (vs. total area) |
|---|---|---|---|
| HMB containing by-products and DHMB | 9.3 | 10.6 | 72.2 |
| After addition of sodium hypochlorite | 9.0 | 0.1 | 83.2 |
| After addition of calcium hydroxide and filtration of recipitates (highly pure HMB) | 0.4 | 0.3 | 98.0 |
| Highly pure calcium salt of HMB | 0.2 | 0.0 | 99.8 |

<Example 10> (Operation d)

The flasks were charged with 868 g (1.551 mol) of an aqueous solution of sodium hypochlorite having a concentration of 13.3 wt%, cooled, 50.0 g (0.430 mol) of diacetone alcohol was added while maintaining at 0 to 10 °C, the stirring was continued for 30 minutes at 0 to 10 °C to react the diacetone alcohol with sodium hypochlorite. The reaction mixture was acidified (pH 0.9) with 148.8 g (1.469mol) of hydrochloric acid, the reaction mixture was concentrated (concentrated under reduced pressure while maintaining at 50 to 60 °C for 5 hours) until the residue weight was 308 g (about 30 % of the weight before concentration), the HMB of the free acid was extracted and recovered with ethyl acetate (126 ml×3 times), and the three extracts were combined. An aqueous solution of sodium hydroxide was added to this combined solution in the amounts shown in Table 12 below to form DHMB sodium salts, and the respective liquid phases were separated at 20 °C. The quantity of DHMB in the upper layer after the separation was calculated by HPLC measurements according to the condition of Table 1. The results are indicated in the following table 12.

Table 12

Table 12

| | Amount of added sodium hydroxide | DHMB area % (vs. HMB) |
|---|---|---|
| Extracted and recovered HMB with ethyl acetate (Extracted and recovered solution) | Before addition | 17.2 |
| Upper layer after addition of only water | 0 times molar (amount of substance to DHMB) | 16.9 |
| Upper layer after addition of 1 w% sodium hydroxide solution | 0.044 times molar (amount of substance to DHMB) | 16.4 |
| Upper layer after addition of 5 w% sodium hydroxide solution | 0.22 times molar (amount of substance to DHMB) | 14.0 |
| Upper layer after addition of 10 w% sodium hydroxide solution | 0.44 times molar (amount of substance to DHMB) | 11.5 |

<Example 11> (Operation of a, b and d)

The flasks were charged with 868 g (1.551 mol) of an aqueous solution of sodium hypochlorite having a concentration of 13.3 wt%, cooled, 50.0 g (0.430 mol) of diacetone alcohol was added while maintaining at 0 to 10 °C, the stirring was continued for 30 minutes at 0 to 10 °C to react the diacetone alcohol with sodium hypochlorite. The reaction mixture was acidified (pH 1.0) with 147.9 g (1.460 mol) of hydrochloric acid, the reaction mixture was concentrated (concentrated under reduced pressure while maintaining at 50 to 60 °C for 5 hours) until the residue weight was 293 g (about 30 % of the weight before concentration), the HMB of the free acid was extracted and recovered with ethyl acetate (111 ml×3 times), and three extracts were combined. This combined solution was added 8.0 g (0.35-fold molar as the amount of DHMB) of 10 wt% aqueous sodium hydroxide solution to form DHMB sodium salts, and the respective liquid phases were separated at 20 to 30 °C. The upper layer solution obtained by separating was 24.1 g (0.043 mol) of a 13.3 wt% aqueous solution of sodium hypochlorite, and the mixture was stirred at 30 to 31 °C for 3 hours. The stirred mixture solution was added 1.50 g (0.015 mol) of hydrochloric acid to acidify the pH 0.6, followed by 0.446 mol of calcium hydroxide in terms of mass to 1.1 g to form DHMB calcium salts, and the respective liquid phases were separated at 40 °C. The separated upper layer was concentrated to give 0.218 mol of highly pure HMB 25.8g. The amounts of by-products (by-products of the oxidative reactions of DAA by NaClO) and the amounts of DHMB and others in the respective cases were calculated by HPLC measurements according to the condition of Table 1. The results are indicated in the following table 13.

Table 13

Table 13

| | DHMB area % (vs. HMB) | By-products area % (vs. HMB) | HMB area % (vs. total area) |
|---|---|---|---|
| After reaction | 17.8 | 33.3 | 51.6 |
| Extracted and recovered HMB with ethyl acetate (Extracted and recovered solution) | 16.0 | 8.1 | 75.3 |
| Upper layer after addition of 10 w% sodium hydroxide solution | 11.4 | 7.5 | 80.0 |
| HMB (highly pure HMB) obtained by concentrating the upper layer containing calcium hydroxide | 0.3 | 0.0 | 98.1 |

Industrial applicability

The 3-hydroxy-3-methylbutyrate or salt thereof produced by the production method of the present invention can be used as a sports supplement, a locomotive syndrome prevention supplement, a sarcopenia prevention supplement, a metabolic syndrome prevention supplement, or a diet supplement by being formulated in a health food, a specified health food, a functional label food and others.

## Claims

**1.** A method for producing of 3-hydroxy-3-methylbutyrate or salt thereof comprising a preparation step for the preparation of a solution containing 3-hydroxy-3-methylbutyrate and impurities and a treatment step for reducing or eliminating the impurities in the solution, wherein the solution contains a by-products of oxidation of diacetone alcohols with hypochlorous acid or salt thereof and/or 2,3-dihydroxy-3-methylbutanoic acid and 3-hydroxy-3-methylbutyrate and impurities in the solution are exposed to environments of pH6.0 or less, and the treatment step is characterized to perform at least one of the following operations (a) to (d) on the solution.

(a) Hypochlorous acid or salt thereof is added to the solution.
(b) The solution is maintained at 40 to 200 °C for 30 minutes or more, provided that the solution contains hypochlorous acid or salt thereof.
(c) Calcium salt is added to the solution, and the precipitated calcium 2,3-dihydroxy-3-methylbutanoate is separated from the liquid phase in which 3-hydroxy-3-methylbutyrate and/or salt thereof is dissolved.
(d) A base is added to the solution to separate the liquid phase in which the salt of 2,3-dihydroxy-3-methylbutanoic acid formed is dissolved and the liquid phase in which 3-hydroxy-3-methylbutyrate and/or salt thereof is dissolved.

[Formula 1]

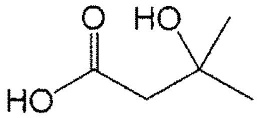

3-hydroxy-3-methylbutyrate
(HMB)

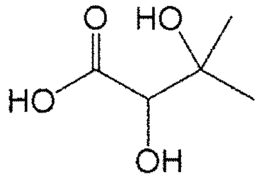

2,3-dihydroxy-3-methylbutanoic acid
(DHMB)

2. The method for producing 3-hydroxy-3-methylbutyrate or salt thereof according to claim 1, wherein the preparation step comprises reacting diacetone alcohol with hypochlorous acid (HClO) or salt thereof (MClO) to form 3-hydroxy-3-methylbutyrate.

[Formula 2]

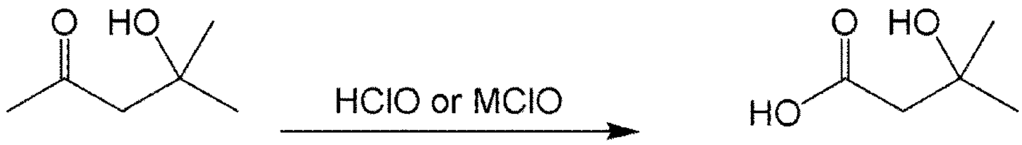


diacetone alcohol
(DAA)

3-hydroxy-3-methylbutyrate
(HMB)

3. The method for producing 3-hydroxy-3-methylbutyrate or salt thereof according to claims 1 or 2, wherein the content of 3-hydroxy-3-methylbutyrate in the solution is 0.01 to 10 mol/l.

4. The method for producing 3-hydroxy-3-methylbutyrate or salt thereof according to any one of claims 1 to 3, wherein the amount of added hypochlorous acid or salt thereof in the operation of (a) is 0.0005 to 10 times as the amount of substance converted to the content of 3-hydroxy-3-methylbutyrate in the solution.

5. The method for producing 3-hydroxy-3-methylbutyrate or salt thereof according to any one of claims 1 to 4, wherein the amount of added calcium salt in the operation (c) is 0.1 to 10 times as the amount of substance converted to the content of 2,3-dihydroxy-3-methylbutanoic acid in the solution.

6. The method for producing 3-hydroxy-3-methylbutyrate or salt thereof according to any one of claims 1 to 5, wherein the amount of added base in the operation (d) is 0.1 to 10 times as the amount of substance converted to the content of 2,3-dihydroxy-3-methylbutanoic acid in the solution.

7. The method for producing 3-hydroxy-3-methylbutyrate or salt thereof according to any one of claims 1 to 6, further comprising a separation step of adding calcium hydroxide to the solution obtained through the treatment step and separating the precipitated calcium 3-hydroxy-3-methylbutanoate from the liquid phase.

Figure 1

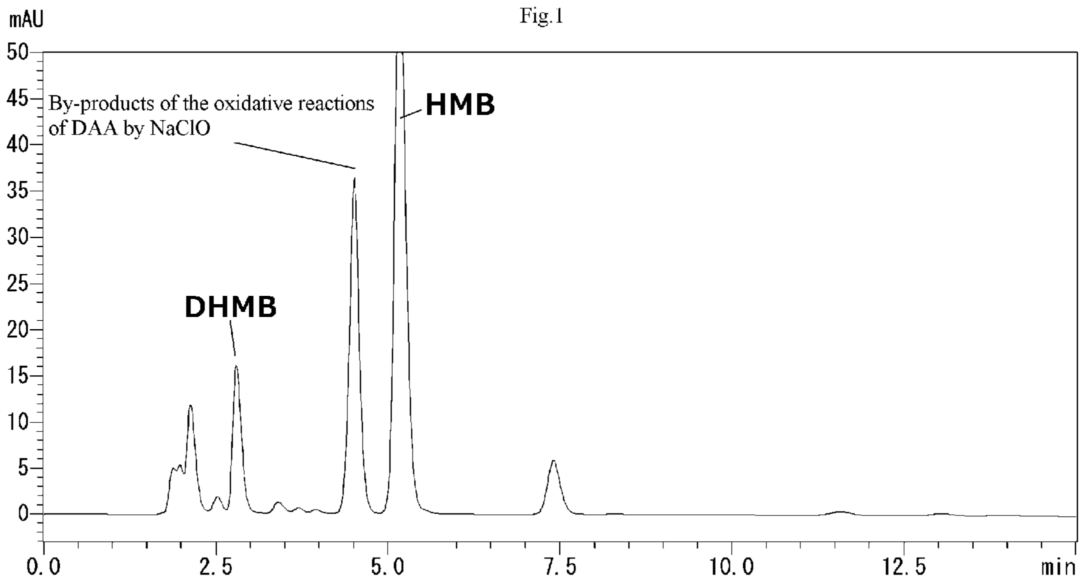


Figure 2

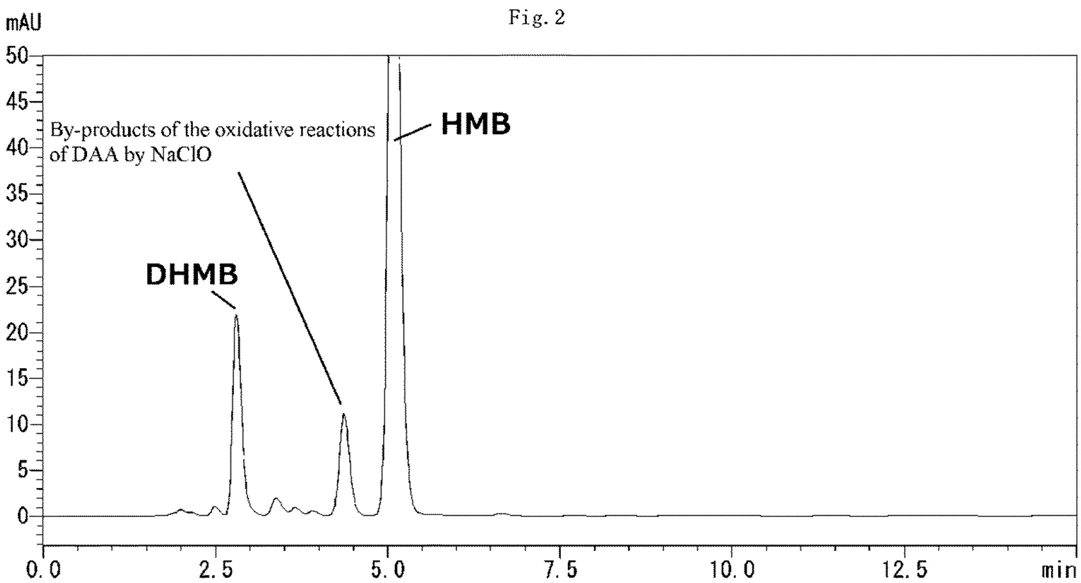

Figure 3

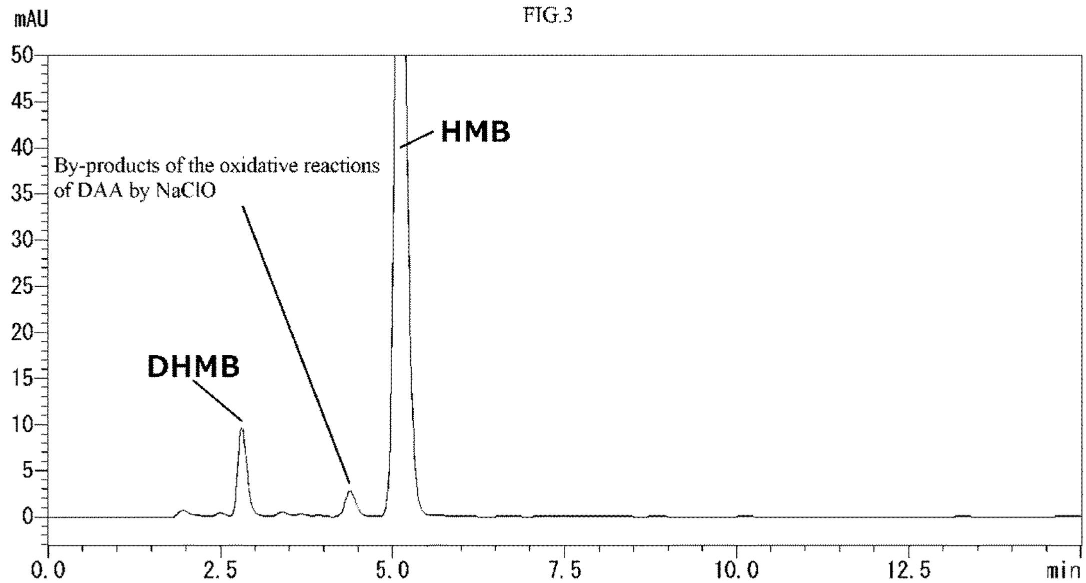
mAU
FIG.3
50
45
40
35
30
25
20
15
10
5
0
By-products of the oxidative reactions of DAA by NaClO
HMB
DHMB
0.0
2.5
5.0
7.5
10.0
12.5
min

## INTERNATIONAL SEARCH REPORT

International application No.
PCT/JP2018/002463

A. CLASSIFICATION OF SUBJECT MATTER
Int.Cl. C07C51/43(2006.01)i, C07C51/48(2006.01)i, C07C59/01(2006.01)i
According to International Patent Classification (IPC) or to both national classification and IPC

B. FIELDS SEARCHED
Minimum documentation searched (classification system followed by classification symbols)
Int.Cl. C07C51/43, C07C51/48, C07C59/01

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN), CASREACT (STN)

C. DOCUMENTS CONSIDERED TO BE RELEVANT

| Category* | Citation of document, with indication, where appropriate, of the relevant passages | Relevant to claim No. |
|---|---|---|
| X | US 4992470 A (IOWA STATE UNIVERSITY RESEARCH FOUNDATION, INC.) 12 February 1991, example 1 (Family: none) | 1-7 |
| A | US 6090978 A (MET-RX USA, INC.) 18 July 2000, claim 1, examples & US 6248922 B1 & US 2001/0014757 A1 | 1-7 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 April 2018 (04.04.2018) | 17 April 2018 (17.04.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/002463

C (Continuation). DOCUMENTS CONSIDERED TO BE RELEVANT

| Category* | Citation of document, with indication, where appropriate, of the relevant passages | Relevant to claim No. |
|---|---|---|
| A | COFFMAN, D. D. et al., "Syntheses by free-radical reactions. V. A new synthesis of carboxylic acids", Journal of the American Chemical Society, 1958, 80, 2882-2887 | 1-7 |
| A | JP 2014-525410 A (ABBOTT LABORATORIES) 29 September 2014, claim 1 & US 2014/0256980 A1, claim 1 & WO 2013/025775 A1 & EP 2744489 A1 & CA 2846041 A & MX 2014001835 A & CN 103857390 A | 1-7 |
| A | JP 2016-514733 A (TSI (CHINA) CO., LTD.) 23 May 2016, claim 1 & US 2016/0052856 A1, claim 1 & WO 2014/166273 A1 & EP 2985275 A1 & CN 104098461 A & CA 2909226 A & AU 2013386219 A | 1-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014525410 A
- JP 2016514733 A